# EUROPEAN PATENT APPLICATION

(11) **EP 4 197 462 A1**
(43) Date of publication of application: **21.06.2023**
(21) Application number: 22209362.7
(22) Date of filing: 24.11.2022
(51) Int. Cl.: A61B 17/32

(54) **IMPLANT FOR THE TREATMENT OF AN URINARY TRACT**

(30) Priority: 15.12.2021 US 202163289842 P
(71) Applicant: Olympus Winter & Ibe GmbH, 22045 Hamburg (DE)
(72) Inventor: Wosnitza, Thomas, 21337 Lüneburg (DE); Jürgens, Thorsten, 21037 Hamburg (DE); Schweinberger, Henning, 21493 Basthorst (DE)
(74) Representative: Hoener, Matthias

(57) **Abstract**

The invention provides an implant for treating the lobus medius (medial lobe) in a simple as well as reliable manner. This is achieved in that an implant (10) for treating a urinary tract has a wire structure. The wire structure has a loop wire (11) expandable into a loop, in which loop at least two mesh wires (12) can be tensioned and the wire structure can be placed around the medial lobe.

## Description

The invention relates to an implant according to claim 1.

Various methods or techniques are known for the treatment of the urinary tract, in particular benign prostatic hyperplasia (BPH). In a minimally invasive, particularly body-sparing treatment of BPH symptoms, a removable implant is temporarily placed in the urethra or in the prostatic portion of the patient's urethra. Such an implant is a wire structure made of a shape memory alloy, such as nitinol. The wire structure is pushed into place by a catheter in a collapsed state, where it unfolds into its predetermined basic structure. This structure, which can be formed from three or four wires, is a basket structure. This basket structure expands the urethra. Due to the expansion of the wire structure against the tissue of the urethra, the tissue of the urethra is denatured in the course of a few days. This denaturation of the tissue occurs due to the ischemic pressure of the individual wires on the cells of the tissue, resulting in decreased or complete lack of blood flow. As a result, the lack of blood flow leads to a lack of oxygen in the cells and eventually to cell death. Within a few days, the tissue can be reduced to such an extent that the urinary flow almost returns to normal. After completion of this treatment, the implant can be retrieved from the urethra by means of a catheter.

The technique described above is a very efficient way of treating the tissue in the urethra. The enlargement of the prostate can also cause the lobus medius (medial lobe) to bulge far into the interior of the bladder. Due to this protrusion of the medial lobe, but also due to a possible enlargement of the medial lobe, it is conceivable that the bladder outlet becomes displaced. However, this displacement of the bladder outlet by a protrusion and/or by an enlargement of the medial lobe can also occur independently of an enlargement of the prostate. It is important to avoid obstruction of the bladder outlet for medical reasons. However, known methods and techniques for this are rather complex and traumatizing for the patient. The technique described above is also not suitable for treating the urinary tract accordingly.

On this basis, the invention is based on the task of creating an implant with which the *lobus medius* (medial lobe) can be treated in a simple and reliable manner.

An implant for solving this problem has the features of claim 1. Accordingly, it is provided that an implant for treating a urinary tract has a wire structure. The treatment is carried out by applying a local ischemic pressure to the tissue of a urinary organ, in particular the medial lobe and possibly simultaneously the urethra. This implant is insertable in a folded state with a distal end first into the urethra, where it unfolds to treat the tissue. There, pressure is applied to the tissue by the individual wires of the wire structure. The invention contemplates the wire structure having a loop wire expandable into a loop, at least two mesh wires being tensionable in said loop, and the wire structure being capable of being wrapped around the medial lobe. When unfolded, the implant takes the form of a tennis racket, with the loop wire forming the frame of the racket and the mesh wires forming the covering. To treat the medial lobe, this loop wire is passed around the medial lobe with at least one end of the net wires held loosely. In the process, the tennis racket shape transforms into a trap net-like shape. Once the implant or loop formed by the loop wire is passed over the medial lobe, both the loop wire and the net wires are contracted, causing the loop circumference to decrease and the net wires to tighten. This contraction causes both the loop wire and the mesh wires to exert ischemic pressure on the tissue, causing it to denature within a few days. Following the treatment, the loop can be reopened and removed from the medial lobe. This minimally invasive procedure allows the medial lobe to be treated in a particularly simple, efficient and gentle way.

Preferably, it is provided that a plurality of mesh wires can be tensioned within the loop, wherein the mesh wires are aligned parallel and/or transversely, in particular perpendicularly, to each other. By using multiple mesh wires, for example 2, 3, 4, 5 or 6, multiple pressure points can be created on the medial lobe, resulting in an even more efficient application of the implant. This increase in pressure area allows a greater amount of tissue to be denatured in the same period of time. By alternating the orientation of the mesh wires within the loop, the stability of the loop can be increased. In addition, by arranging the mesh wires at an angle, the tissue can be subjected to pressure over its entire area.

Another preferred embodiment of the invention may provide that one end of the mesh wires as well as the loop wire are fixedly attached to the wire structure and another end of the mesh wires and the loop wire are loosely as well as tensionably guided to the proximal end of the implant where they are connected, in particular detachably connected, to a handling device. The ends can be attached, for example, by clamping, gluing, crimping, knotting or the like. The loose ends can, for example, be brought together to form a wire for handling, or are passed individually through the urethra to the outside. It is also conceivable that the loose ends are movable by means of the handling device. By pulling on the loose ends of the wires, they can be tensioned. By releasing the wires, the loop structure or the mesh structure can be abandoned, allowing the implant to be easily removed from the medial lobe.

It may be envisaged that the handling device is a shaft, a wire a thread or the like. Depending on the application, the implant can first be inserted into the body via this handling device and positioned there. Thereafter, it is conceivable that the handling device can be decoupled from the implant. For salvage and/or readjustment or retightening of the wires, the handling device can be coupled again to the proximal end of the implant. Similarly, it is conceivable that the handling device is permanently connected to the implant.

A particularly advantageous embodiment of the invention may provide that one or more connecting springs are drawn on the loop wire at least in sections, and the mesh wires are threaded through these connecting springs to fix the position. Thus, one embodiment provides that the loop wire forming the loop is completely passed through a guide spring and the net wires are passed through this spring, preferably symmetrically, at opposite positions of the loop to form the tennis racket-like shape. The connecting springs are such that they can receive the loop wire straight. An alternative embodiment provides for the loop wire to have short connecting springs only in sections. These short sections are fixed in position on the loop wire so that the net wires, which are guided by the connecting springs, remain in their predetermined position and do not slip out of place. When the loop wire is spread out to form the loop, the connecting spring follows the increase in the loop circumference due to its property as a spring. Similarly, when the loop wire is contracted, the connecting spring contracts so that it can also be pulled back into the catheter. During treatment, the individual wire sections of the connecting spring have a reinforcing effect on the denaturation of the tissue.

Furthermore, it can be provided in accordance with the invention that the loop wire is designed as a connecting spring. In this embodiment, the loop wire is missing. For the tightening of the loop, a corresponding tightening of the connecting springs takes place. Also, the connecting spring may be made of a shape memory material for the technique described above.

A preferred embodiment of the present invention is explained in more detail below with reference to the drawing. In this show:
- Fig. 1: a schematic representation of an embodiment of an implant, and
- Fig. 2: a schematic side view of the embodiment example according to Fig. 1.

In the figures, a possible embodiment example of the invention is schematically illustrated. It should be expressly noted that the invention is not intended to be limited to this embodiment example. Rather, it is intended that the invention can also be realized by other embodiments.

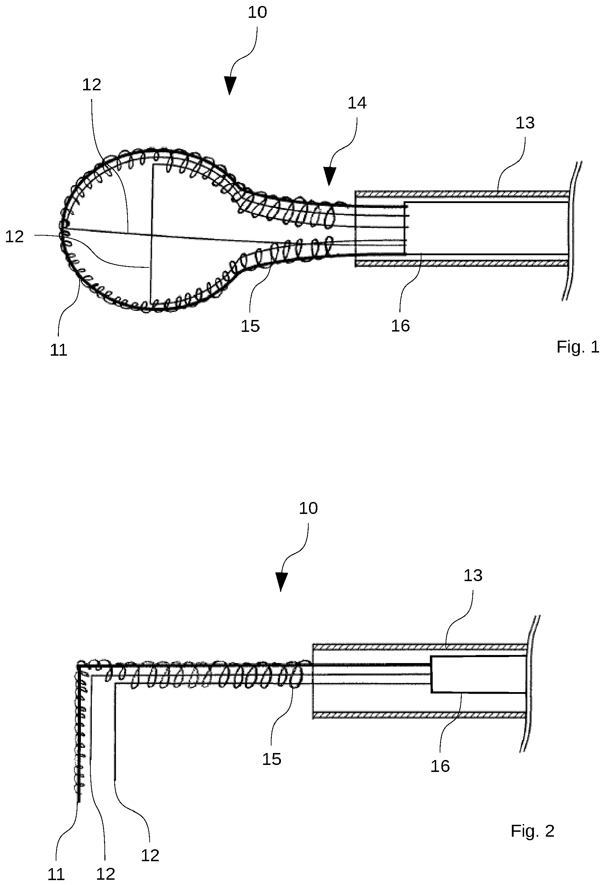

According to the schematic embodiment example of the implant 10 according to the invention shown in Figs. 1 and 2, this is formed by a circumferential loop wire 11 forming a loop. At least two net wires 12 are tensioned in this loop or in this loop wire 11. These net wires 12 are arranged within the loop like the sides of a tennis racket. Ideally, the two net wires 12 are oriented transversely or perpendicularly to each other within the curved loop wire 11.

In the embodiment example shown in Fig. 1, the loop formed by the loop wire 11 is traversed by a net wire 12, with two further net wires 12 positioned perpendicularly thereto. Furthermore, it is conceivable that further net wires 12 are arranged parallel to these net wires 12 within the loop wire 11.

The implant 10 is inserted into the urinary tract through a catheter 13 shown only schematically. The loop wire 11 and the mesh wires 12 are initially folded together within the catheter 13. The loop wire 11 unfolds into the loop by being pushed out of the catheter 13, whereby the mesh wires 12 are tensioned within the loop. This unfolding is realized by the choice of material of the loop wire 11. Namely, the loop wire 11 is preferably made of a shape memory material, for example nitinol. Both the loop wire 11 and net wires 12 may be attachable or detachably attachable to a handling device 16 by at least one of their ends. This handling device 16 may be a rod, a shaft, a wire or a thread. By means of this handling device 16, the implant 10 can be inserted into the body and pulled out again. It is conceivable that the handling device 16 can be releasably coupled to the implant 10. During treatment, the handling device 16 can thus be detached from the implant 14. In particular for salvaging the implant 10, this can be coupled again to the handling device 16.

One end each of the loop wire 11 and the mesh wires 12 are fixed to a proximal end 14 of the implant 10, in particular to the handling device 16. The second ends are loosely returned to the proximal end 14 of the implant 10. There, they can be moved back and forth by pulling and are preferably lockable or lockable to the handling device 16. By exerting traction, the circumference of the loop wire 11 can be reduced, for example. Furthermore, by simultaneously pulling on the free ends of the net wires 12, the mechanical tension of the net wires 12 can be maintained. To provide sufficient mechanical stability, the net wires 12 are particularly made of titanium or a spring steel.

In order to fix the net wires 12 within the loop of the loop wire 11, the net wires 12 are guided along an inner side of the loop wire 11 and are held in position there by fixing means. Thus, the net wires 12 maintain their relative position to each other as the loop diameter is reduced and increased. In an advantageous embodiment of the invention, the loop wire 11 is enclosed by a connecting spring 15. This connecting spring 15 may extend either along the entire length of the loop wire 11 or only in sections.

In the embodiment example shown in Figs. 1, 2, the connecting spring 15 extends over the entire length of the loop wire 11. The individual turns of the connecting spring 15 serve to thread the net wires 12 therethrough. In this way, the net wires 12 maintain their relative position to each other in a simple and reliable manner. The plurality of coils in the connecting springs 15 also allows a plurality of net wires 12 to be tensioned in the loop in virtually any orientation and constellation. It has been shown, however, that even for reasons of space, two to four net wires 12 within the loop achieve a particularly advantageous effect on the fabric.

To treat the medial lobe, the loop formed by the loop wire 11 is passed over the medial lobe. Initially, the loose end of the mesh wires 12 is not fixed. As a result, the net wires 12 and also the loop wire 11 wrap around the medial lobe. In the next step, both the loop wire 11 and the net wires 12 are then pulled together. This changes both the circumference of the loop and the mechanical tension of the mesh wires 12 on the encompassed tissue. During the next few days, the already circumscribed denaturation of the tissue of the medial lobe takes place. At the end of the treatment, the loose end of the loop wire 11 and the mesh wires 12 are released and lifted off the medial lobe. By withdrawing the implant 10 or by retracting the mesh wires 12, they hang out of the plane of the loop with their loose ends as shown in Fig. 2. In this state, the implant 10 can be easily removed from the urinary tract without damaging the body.

### List of Reference Numerals:

- 10: Implant
- 11: Loop wire
- 12: Mesh wire
- 13: Catheter
- 14: Proximal end
- 15: Connecting spring
- 16: Handling device

## Claims

1. An implant (10), in particular a removable implant (10), for the treatment of an urinary tract of a person by applying a local ischemic pressure to the tissue of a medial lobe through a wire structure, wherein the implant (10) is insertable in a folded state with a distal end leading into the urethra and unfolds in the urethra to the wire structure for treating the tissue, **characterized in that** the wire structure comprises a loop wire (11) expandable into a loop, in which loop at least two mesh wires (12) are tensionable and the wire structure is adapted to be wrapped around the medial lobe.

2. Implant (10) according to claim 1, **characterized in that** a plurality of mesh wires (12) can be tensioned within the loop, the mesh wires (12) being aligned parallel and/or transversely, in particular perpendicularly, to one another.

3. Implant (10) according to claim 1 or 2, **characterized in that** one end of the mesh wires (12) and of the loop wire (11) are fixedly attached to the wire structure and another end is loosely as well as tensionably guided to the proximal end (14) of the implant (10) and there connected, in particular detachably connected, to a handling device (16).

4. Implant (10) according to claim 3, **characterized in that** the handling device (16) can be a shaft, a wire a thread or the like.

5. Implant (10) according to one of the previous claims, **characterized in that** the length of the loop wire (11) and the mesh wires (12) are variable so as to exert a pressure on the tissue and release the tissue.

6. Implant (10) according to one of the previous claims, **characterized in that** one or more connecting springs (15) are drawn on the loop wire (11) at least in sections and the mesh wires (12) are threaded through the connecting springs (15) to fix the position.

7. Implant (10) according to one of the previous claims, **characterized in that** the loop wire (11) is formed as a connecting spring (15).

8. Implant (10) according to one of the previous claims, **characterized in that** all wires (11, 12) are brought together at a proximal end (14) of the implant (10), preferably at a crimping point, and the loose wires (11, 12) can be fixed there or guided outwards through the urethra.
